# EUROPEAN PATENT SPECIFICATION

(11) **EP 1 798 243 B1**
(45) Date of publication and mention of the grant of the patent: **11.08.2010**
(21) Application number: 06076643.3
(22) Date of filing: 08.04.2002
(51) Int. Cl.: C07K 16/42, A61K 39/395, A61P 35/00, A61P 35/04

(54) **Ganglioside-associated recombinant antibodies and the use thereof in the diagnosis and treatment of tumors**
Ganglioside-assozierte rekombinante Antikörper und deren Gebrauch fuer Diagnose und Therapien von Tumoren
Anticorps recombinants associés aux gangliosides et leur utilisation dans le diagnostic et le traitement des tumeurs

(30) Priority: 06.04.2001 CU 842001
(43) Date of publication of application: 20.06.2007
(62) Divisional of application: 02759752.5
(73) Proprietor: Centro de Inmunologia Molecular, Ciudad Habana 12100 (CU)
(72) Inventor: Mateo De Acosta Del Rio, Cristina Maria, Boyeros Ciudad de la Habana 10800 (CU); Lombardero Valladares, J., 10 de Octubre Ciudad de la Habana 10500 (CU); Roque Navarro, Lourdes Tatiana, Playa Ciudad de la Habana 11300 (CU); Lopez Requena, Alejandro, Avenida de Acosta n° 210(bajos), Ciudad de la Habana 10500 (CU)
(74) Representative: van Loon, C.J.J.

(56) References cited:
- EP-A1- 0 657 471
- CA-A1- 2 308 142
- VAZQUEZ ANA MARIA ET AL: "Antitumor properties of an anti-idiotypic monoclonal antibody in relation to N-glycolyl-containing gangliosides" ONCOLOGY REPORTS, vol. 7, no. 4, July 2000 (2000-07), pages 751-756, XP009084777 ISSN: 1021-335X
- VAZQUEZ A M ET AL: "Syngeneic anti-idiotypic monoclonal antibodies to an anti-NeuGc-containing ganglioside monoclonal antibody" HYBRIDOMA, vol. 17, no. 6, December 1998 (1998-12), pages 527-534, XP009084778 ISSN: 0272-457X
- ALFONSO MAURO ET AL: "An anti-idiotype vaccine elicits a specific response to N-glycolyl sialic acid residues of glycoconjugates in melanoma patients" JOURNAL OF IMMUNOLOGY, vol. 168, no. 5, 1 March 2002 (2002-03-01), pages 2523-2529, XP009084775 ISSN: 0022-1767
- PEREZ A ET AL: "Immunogenetic analysis of variable regions encoding AB1 and gamma-Type AB2 antibodies from the NeuGc-containing ganglioside family" HYBRIDOMA, LIEBERT, NEW YORK, NY, US, vol. 20, no. 4, 2001, pages 211-221, XP002902755 ISSN: 0272-457X
- VAUGHAN T J ET AL: "HUMAN ANTIBODIES BY DESIGN" NATURE BIOTECHNOLOGY, NATURE PUB. CO, NEW YORK, NY, US, vol. 16, no. 6, June 1998 (1998-06), pages 535-539, XP000941675 ISSN: 1087-0156
- WINTER G ET AL: "ANTIBODY-BASED THERAPY, HUMANIZED ANTIBODIES" TRENDS IN PHARMACOLOGICAL SCIENCES, ELSEVIER, HAYWARTH, GB, vol. 14, no. 5, May 1993 (1993-05), pages 139-143, XP001205172 ISSN: 0165-6147
- LOPEZ-REQUENA A. ET AL.: "Chimeric Anti-N-Glycolyl-Glanglioside and Its Anti-Idiotypic MABs: Immunodominance fo Their Variable Regions" HYBRIDOMA AND HYBRIDOMICS, vol. 22, no. 4, 2003,

## Description

### Technical Field

The present invention is related with biotechnology; in particular with new antibodies obtained by genetic engineering, specifically with chimeric and humanized antibodies obtained from the anti-idiotype murine monoclonal antibody 1E10 (MAbai 1E10), that recognizes the murine MAb P3.

More specifically, the invention is related with antibodies that bind to gangliosides containing N-glycolylated sialic acid, but not with the acetylated forms of the gangliosides neither with neuter glycolipids. Gangliosides containing N-glycolylated sialic acid are antigens widely expressed in breast cancer and melanomas; the anti-tumor effect of the MAbai 1E10 has also been demonstrated in experimental models.

The present invention is also related with the pharmaceutical compositions that contain the previously described recombinant antibodies useful in the diagnosis and therapy of the cancer.

### Prior Art

Gangliosides are glycosphingolipids that contain sialic acid and they are present in the plasmatic membrane of cells of vertebrates (Stults and collaborators (1989): Glycosphingolipids: structure, biological source and properties, Methods Enzymology, 179:167-214). Some of these molecules have been reported in the literature like antigens associated to tumors or tumor markers (Hakomori and collaborators (1991): Possible functions of tumor associated carbohydrate antigens, Curr. Opin. Immunol., 3: 646-653), for that reason the use of anti-gangliosides antibodies has been described like useful in the diagnosis and therapy of cancer (Hougton and collaborators (1985): Mouse monoclonal antibody IgG3 antibody detecting GD3 ganglioside: to phase I trial in patients with malignant melanoma, PNAS USA, 82:1242-1246; Zhang and collaborators (1997): Selection of carbohydrate tumor antigens as targets for immune attack using immunohistochemistry. I. Focus on gangliosides, Int. J. Cancer, 73: 42-49). Sialic acid more frequently expressed in animals are N-acetyl (NeuAc) and N-glycolyl (NeuGc) (Corfield et al. (1982): Occurence of sialic acids, Cell. Biol. Monogr., 10: 5-50). NeuGc is not expressed in normal human and chickens tissues, but it is broadly distributed in other vertebrates (Leeden et Yu (1976): Chemistry and analysis of sialic acid. In: Biological Role of Sialic Acid. Rosemberg A and Shengtrund CL (Eds). Plenum Press, New York, 1-48; Kawai et collaborators (1991): Quantitative determination of N-glycolylneuraminic acid expression in human cancerous tissues and avian lymphoma cell lines as a tumor associated sialic acid by gas chromatography-mass spectrometry, Cancer Research, 51: 1242-1246). However, there are reports that show that antibodies anti-NeuGc, recognize some human tumors and tumor cell lines (Higashi et colaboradores (1988): Detection of gangliosides as N-glycolylneuraminic acid specific tumor-associated Hanganutziu-Deicher antigen in human retinoblastoma cells, Jpn. J. Cancer Res., 79: 952-956; Fukui et collaborators, (1989): Detection of glycoproteins as tumor associated Hanganutziu-Deicher antigen in human gastric cancer cell line, NUGC4, Biochem. Biophys. Res. Commun., 160: 1149-1154). Increased levels of GM3 (NeuGc) gangliosides have been found in human breast cancer (Marquina et collaborators (1996): Gangliosides expressed in human breast cancer, Cancer Research, 1996; 56: 5165-5171), this result makes attractive the use of this molecule as target for cancer therapy.

The Mab P3 is a murine monoclonal antibody with IgM isotype, that was obtained when fusing murine splenocites from a BALB/c mouse immunized with liposomes containing GM3(NeuGc) and tetanic toxoid, with the cell line P3-X63-Ag8.653; which is a murine myeloma. This Mab P3 reacts strongly with gangliosides containing N-glycolylated sialic acid but not with the acetylated forms of the gangliosides neither with the neuter glycolipids. It was demonstrated by immunocitochemestry and immunohistochemestry studies carried out with cell lines and tissues from benign and neoplasic tumors that the AcM P3 recognizes breast cancer (Vázquez et collaborators (1995): Generation of a murine monoclonal antibody specific for N-glycolylneuraminic acid-containing gangliosides that also recognizes sulfated glycolipids, Hybridoma, 14: 551-556) and melanoma. The MAb P3 induced anti-idiotypic immune response (Ab2) in mice BALB/c (syngeneic model), even without adjuvant and carrier protein, (Vázquez et collaborators (1998): Syngeneic anti-idiotypic monoclonal antibodies to an anti-NeuGc-containing ganglioside monoclonal antibody, Hybridoma, 17: 527-534). The role of the electronegative groups, sialic acid (for gangliosides) and SO₃- (for sulfatides), in the recognition properties of this antibody was suggested by immunochemical analysis (Moreno et collaborators (1998): Delineation of epitope recognized by an antibody specific for N-glycolylneuraminic acid-containing gangliosides, Glycobiology, 8: 695-705).

The anti-idiotypic MAb 1E10 (IgG1) was obtained from a mouse BALB/c immunized with the MAb P3 coupled to KLH. Mabai 1E10 recognized specifically the MAb P3 and it did not bind other IgM anti-ganglioside antibodies. Moreover, Mabai 1E10 inhibited the specific binding of MAb P3 to the GM3(NeuGc) and to a cell line MDA-MB-435, derived from ductal breast carcinoma (positive for MAb P3 binding). The MAbai 1E10 induced a strong immune response of Ab3 antibodies when mice from syngeneic or alognic models were immunized, these Ab3 antibodies didn't exhibit the same specificity as the MAb P3 eve when they carry idiotopes similar to those carried by the Ab1 antibody (Vázquez et collaborators (1998): Syngeneic anti-idiotypic monoclonal antibodies to an anti-NeuGc-containing ganglioside monoclonal antibody, Hybridoma, 17: 527-534). MAbai 1E10 induced a strong antitumor effect in syngeneic as well as alogenic mice. The growth of the mammary carcinoma cell line F3II was significantly reduced by the repeated dose of the MAbai 1E10 coupled KLH in Freund's adjuvant, when BALB/c mice were vaccinated. Also the number of spontaneous lung metastasis was reduced after the vaccination. The intravenous administration of the Mabai 1E10 to C57BU6 mice inoculated, 10 to 14 days after the intravenous inoculation of melanoma cells B16, caused a dramatic reduction of the number of lung metastases when compared with mice treated with an irrelevant IgG. These results suggest that more than one mechanism of antitumor effect is triggered (Vázquez et collaborators (2000): Antitumor properties of an anti-idiotypic monoclonal antibody in relation to N-glycolyl-containing gangliosides, Oncol. Rep., 7: 751-756, 2000).CA 2308142 discloses the murine anti-idiotypic monoclonal antibody 1E10 which recognizes the monoclonal anti-ganglioside antibody P3.

Even when hybridoma technology has been developed during 15 years (Koehler et Milstein (1975): Continuous cultures of fused cells secreting antibody of predefined specificity, Nature, 256: 495-497) and when monoclonal antibodies are still very useful in diagnosis as well as research they have not demonstrated their therapeutic effectiveness in human. It has been mainly due to their short half-life in blood and to that murine effector functions fail for the human immune system, and also for the human anti-mouse antibody immune response (HAMA response). Otherwise, genetic engineering technology has revolutionized the potential of the MAb utility, since manipulating immunoglobulin genes it is possible to obtain modified antibodies with reduced antigenicity, as well as to improve its effector functions for the treatment or diagnosis of certain pathologies. Methods for reduce immunoglobulin immunogenicity have as essential objective to diminish the differences between a murine antibody and a human immunoglobulin, without altering the antigen recognition specificity (Morrison et Oi (1989): Genetically engineered antibody molecules, Adv immune)., 44: 65-92).

Recently several methods have been developed to humanize murine or rat antibodies and this way to reduce the xenogenic immune response against foreign proteins when they are injected in human. One of the first approach to reduce the antigenicity were the chimeric antibodies, in which the variable domains of the murine protein are inserted in constant domains of human molecules, that exhibit the same specificity but reduced immunogenicity compared to their murine counterparts, human effector functions are preserved by chimeric antibodies, (Morrison et collaborators (1984): Chimeric human antibody molecules: Mouse antigen-binding domains with human constant region domains, PNAS USA, 81: 6851-6855). Even when chimeric antibodies have the same specificity as the murine counterpart, an immune response to the rodent variable regions is frequently observed.

In an attempt to further reduce the immunogenicity of chimeric antibodies, only the CDRs from the rodent monoclonal antibody have been grafted onto human framework regions and this hybrid variable region expressed with human constant regions (Jones et collaborators (1986): Replacing the complementary-determining regions in a human antibody with those from a mouse, Nature 321: 522-524; Verhoeyen et collaborators (1988): Reshaping human antibodies: grafting an antilysozyme activity, Science 239, 1534-1536). However, this approach has several shortcomings: frequently the resulting antibody has decreased affinity and a number of framework residues must be backmutated to the corresponding murine ones to restore binding (Rietchmann et collaborators (1988): Reshaping human antibodies for therapy, Nature, 332: 323-327; Queen et collaborators (1989): A humanized antibody that binds to the interleukin 2 receptor, PNAS USA, 86: 10029-10033; Tempest et collaborators (1991): Reshaping a human monoclonal antibody to inhibit human respiratory syncytial virus infection in vivo, Biotechnology, 9: 266-272). In addition, persisting immunogenicity is frequently seen in the CDR-grafted antibodies.

Mateo and collaborators (US Patent Number US 5 712 120) have described a procedure in order to reduce immunogenicity of murine antibodies. According to the method, the modifications are restricted to the variable domains and specifically to the murine FRs of chimeric antibodies. Moreover, the replacements are only carried out in those regions of the FRs that have amphipatic sequences and therefore they are potential epitopos recognized by T cells T. The method comprises judiciously replacement of few amino acid residues, located in the potential immunogenic epitopes by the corresponding residues from the most homologous human sequence, the amino acids which are mainly responsible for canonical structures and also the residues in the immediate neighbourhood of the CDRs or into the Vernier zone, must be retained. The resulting antibody retains its antigen binding specificity and to be less immunogenic than either its murine or chimeric predecessor (Mateo et collaborators (2000): Removal of T cell epitopes from genetically engineered antibodies: Production of modified immunoglobulins with reduced immunogenicity, Hybridoma 19: 463-71), these properties increases their therapeutic utility. Using this new procedure only few mutations, and of course less genetic manipulations, have to be done.

### Detailed description of the Invention

The present invention is related to recombinant antibodies, obtained by genetic engineering technology.

The invention is related with a chimeric antibody derived from the murine monoclonal antibody 1E10 produced by the hybridoma cell line with deposit number ECACC 97112901, and it is an antidiotype antibody, which recognizes the MAb P3. The MAbai 1E10 is characterized by the following sequences of the hypervariable regions (CDRs) of the heavy and light chains:

### HEAVY CHAIN

CDR1:SYDIN
CDR2: WIFPGDGSTKYNEKFKG
CDR3:EDYYDNSYYFDY

### UGHT CHAIN

CDR1:RASQDISNYLN
CDR2: YTSRLHSG
CDR3: QQGNTLPWT
and wherein the FRs sequences of the heavy and light chain are the following:

### HEAVY CHAIN

FR1: QVQLQQSGAELVKPGASVKLSCKASGYTFT
FR2: WVRQRPEQGLEWIG
FR3: KATLTTDKSSSTAYMQLSRLTSEDSAVYFCAR
FR4: WGQGTTLTV

### LIGHT CHAIN

FR1:DIQMTQTTSSLSASLGDRVTISC
FR2: WYQQKPDGTVKLLIY
FR3: VPSRFSGSGSGTDYSLTISNLEQEDIATYFC
FR4: FGGGTKLESK

The chimeric antibody of the present invention, contains the constant region of heavy chain human IgG1 and the constant region of light chain human Ck. In another aspect, the present invention is related with a humanized antibody derived from the Mab 1E10 produced by the hybridoma cell line with deposit number ECACC 97112901, characterized because it contains the constant region of human heavy chain IgG1 and the constant region of human light chain Ck and FRs regions of the heavy and light chain contains any of the following point mutations:

### LIGHT CHAIN:

Position 7: Thr by Ser
Position 8: Thr by Pro
Position 15: Leu by Val

### HEAVY CHAIN:

Position 5: Gln by Val
Position 40: Arg by Ala
Position 42: Glu by Gly
Position 87 (83 according Kabat's numbering): Thr by Arg

In another aspect, the present invention is related with the cell lines that express the described chimeric and humanized antibodies; additionally the invention is related with pharmaceutical compositions comprising the described antibodies.

Preferably it is related with pharmaceutical compositions for use in the treatment of breast, lung, digestive system, urogenital system, melanomas, sarcomas and neuroectodermic tumors, their metastases and relapses, comprising the described antibodies and an appropriate exicipient.

In another representation of the present invention, the pharmaceutical compositions can be used for the localization and diagnosis *in vivo* of breast, lung, digestive system urogenital system, melanomas, sarcomas and neuroectodérmico tumors, their metastases and relapses, comprising the described antibodies.

### cDNA Synthesis and Gene Amplification by PCR (Polymerase chain reaction) of the variable region of MAb P3 and Mabai 1 E10.

Cytoplasmic RNA was extracted from about 10⁶ hybridoma cells of P3 (murine IgM MAb, that recognizes to the GM3 N-glycolylated ganglioside) or of 1E10 (antidiotype anti-P3 antibody). The RNA was extracted by using the reagent TRIZOL (GIBCO BRL, Grand Island, NY), according to the maker's instructions.

The cDNA synthesis reaction was carried out mixing 5µg of the RNA, 25 pmoles of Vh (complementary to the constant region of murine IgM for VHP3, and with the constant region of murine IgG1 for VH 1E10) or Vk (complementary to constant murine kappa region for both antibodies), 2.5 mM of each dNTPs, 50 mM Tris-Hcl pH 7.5, 75 mM KCI, 10 mM DTT, 8 mM MgCI2 and 15 units of RNAsa inhibitor in a 50 ul reaction mixture. It was heated at 70°C, for 10 minutes and slowly cooled up to 37°C. Then, 100 units of MLV reverse transcriptase enzyme were added and the incubation at 42°C continued for one hour.

The variable regions VK and VH cDNAs were amplified by PCR. Shortly, 5 ul cADN of VH or VK were mixed with 25 pmoles of specific primers, 2.5 mM of each dNTP, 5 ul constituents of 10X buffer Taq DNA polimerasa and 1 unit of this enzyme. The samples were subjected to 25 thermal cycles at 94°C, 30 sec; 50°C, 30 sec; 72°C, 1 min, and a last incubation for 5 minutes at 72°C.

### Cloning and Sequencing of Amplified cDNA

The PCRs products of VH and VK (of the P3 and of the 1E10 respectively) were cloned into TA vector (TA Cloning kit. Promega, USES). The resulting clones were sequenced by the dideoxy method using T7 DNA Polymerase (T7 sequencing kit, Pharmacia, Sweden).

### Construction of chimeric genes

The VH VK genes were excised from TA vectors by enzymatic digestion and they were cloned into the respective expression vectors (Coloma et collaborators (1992): Novel vectors for the expression of antibody molecules using variable regions generated by polymerase chain reaction, J. Immunol. Meth., 152: 89-104).

The VH gene was excised from the TA vector by enzymatic digestion with EcoRV and Nhel and cloned in an expression vector (PAH 4604) that has included a variable region human IgG1 and the histidinol resistance gene. The resultant constructions were P3VH-PAH4604 and 1E10VH-PAH4604. The VK genes were excised from TA vector by enzymatic digestion with EcoRV and SALI and cloned in an expression vector (PAG4622). This vector has included mycophenolic resistance gene and the human kappa constant region. The resultant constructions were P3VK-PAG4622 and 1E10VK-PAG4622.

### Chimeric antibodies expression

NS-0 Cells were electroporated with 10 µg of P3VK-PAG4622 or 1E10VK-PAG4622, clones expressing human kappa light chains were transfected with 10 ug of P3VH-PAH4604 or 1E10VH-PAH4604.

The DNAs were linearized by digestion with Pvul enzyme, precipitated with ethanol and dissolved in 50 ul of PBS. Approximately 10⁷ cells were harvested by centrifugation and resupended in 0.5 ml of PBS together with the digested DNA in an electroporation cuvette. After 10 minutes on ice, the cells were given a pulse of 200 volts and 960 uF and left in ice for a further 10 minutes. The cells were distributed into 96 wells plate with D'MEM F12 plus 10% fetal calf serum. Two or four days later, it is added selective medium (D'MEM F12 with mycophenolic acid 0,45 ug/mL or histidinol 10mM, respectively). Transfected clones were visible with the naked eyes 14 days later. The presence of human antibody in the medium of wells containing transfected clones was measured by ELISA. Microtiter plate wells were coated with goat anti-human kappa light chain (for human kappa chain producing clones) or anti-human IgG (gamma chain specific) (for the complete antibody producing clones) antibodies. After washing with PBST (saline phosphate buffered solution containing 0.05% Tween 20), diluted culture medium of the wells containing transfectants was added to each Microtiter well for one hour at 37°C. The wells were washed with PBS-T and peroxidase of spicy radish-conjugated goat anti-human kappa light chain or alkaline fosfatase-conjugated goat anti-human IgG (gamma chain specific), were added and incubated at 37°C one hour. The wells were washed with PBS-T and substrate buffer containing *o-*phenylendiamine or *p*-nitrophenylphosphate, respectively, was added. After half hour absorbance at 492 or 405 nm respectively, was measured.

### Construction of the humanized antibodies P3hu and 1E10 hu by humanization of T cell epitopes

### Prediction of T cell epitopes

The sequences of P3 and 1E10 variable domains were analysed with the algorithm AMPHI (Margalit et collaborators (1987): Prediction of immunodominant helper T cell antigenic sites from the primary sequence, J. Immunol., 138: 2213-2229). It searched helical amphipatic segments, with 7 or 11 aminoacid residues, which have been associated with T immunogenicity. The program SOHHA also predicted helical hydrophobic segments. (Elliot et collaborators (1987) An hypothesis on the binding of an amphipatic, alfa helical sequence in li to the desotope of class II antigen, J.Immunol., 138: 2949-2952). Both algorithms predicted which segments from variable region sequences of antibodies P3 and 1E10 could be presented to T-helper cells in the context of MHC class II molecules.

### Sequences homology searching

The aminoacid sequences of murine variable regions were compared with the immunoglobulin sequences included in the GeneBank and EMBL database (availables in Internet). The most homologous human variable region sequence was determined for each antibody. Software PC-TWO HIBIO PROSIS 06-00 (Hitachi) was used for sequences homology searching.

### Analysis for the immunogenicity reduction

The aim of the method is to reduce immunogenicity breaking or humanizing potential immunogenic T epitopes, with a minimum of changes. The method comprises judiciously replacement of few amino acid residues, located into helical amphipatic segments. The amino acids, which are mainly responsible for canonical structures and also the residues in the immediate neighbourhood of the CDRs or into Vernier zone, must be retained.

According to the method, murine variable region sequences were compared with the most homologous human sequence and different aminoacid residues at each position between the murine MAb and the most homologous human sequence were identified, only residues into FRs were taken into account (Kabat (1991), Sequences of proteins of immunological interest, Fifth Edition, National Institute of Health), the previously defined residues were replaced by those residues present in the most homologous human sequence. Replacements were made by directed mutagenesis techniques.

Residues involved in three-dimensional structure of the binding site were not mutated; it could affect antigen recognition. Additional information about the influence of the replacements in the tertiary structure can be obtained by molecular modelling of the antigen binding site.

The presence of proline residues into the helical amphipatic segment and the fact that a certain murine residues doesn't appear in the same position in the most homologous human sequence but be frequent in other human immunoglobulins, must be kept in mind. For that reason there is not a unique ensemble of murine amino acids to be replaced into the frameworks. It is possible to obtain different versions of the modified antibody with different numbers of replacements.

### Cloning and expressing humanized antibodies P3hu and 1E10hu

The genetic constructions corresponding to the P3hu and 1E10hu, were cloned in expression vectors following the method described for the chimeric antibodies. The resultants constructions were P3VKhu-PAG4622 or 1E10Vkhu-PAG4622 and P3VHhu-PAH4604 and 1E10VHhu-PAH4604. They were transfected into NS-0 cells following the protocol described previously for chimeric antibodies.

### Purification of the recombinant antibodies

The recombinant antibodies were purified by affinity chromatography using protein A (Pharmacia, Upssala, Sweden).

### Biological activity

The specific binding to antigen measured by ELISA tested the biological activity of the recombinant antibodies.

For recombinant MAb P3, Microtiter plates were coated with GM3(NeuGc) ganglioside in methanol. After drying one hour at , unspecific binding was blockade with bovine sera albumin (BSA) 1% % in Tris-HCl buffer, incubated for one hour at 37°C. The wells were washed with PBS and incubated for 1 hour at 37°C with purified recombinant Mab P3. The wells were washed with tris-HCl and a goat anti- human antibody conjugated with alkaline fosfatase was added and incubated at 37°C for one hour. Finally, the wells were washed and the substrate buffer containing *p*-nitrophenylphosphate was added. After half hour absorbance at 405 nm respectively, was measured.

For recombinant Mabal 1E10, the ELISA assay was similar, except that wells were coated with Mab P3 and washing were made with PBS-0.05% Tween 20.

### Examples.

In the following examples all the enzymes used, as well as reagents and materials were obtained from commercial sources unless the opposite is specified.

EXAMPLES 1-3 do not concern the invention.

### Example 1. Obtaining of chimeric MAb P3.

The cDNA synthesis was obtained by a reaction with reverse transcriptase enzyme, starting with RNA from the hybridoma producing Mab P3, as described previously. The sequence of the specific primers used in this reaction is shown:
**For VH:**
   5' AGGTCTAGAA(CT)CTCCACACACAGG(AG)(AG)CCAGTGGATAGAC 3'
**For VK:**
   5' GCGTCTAGAACTGGATGGTGGGAAGATGG 3'
   cDNA VHP3 and cDNA VKP3 were amplified by PCR using Taq Pol and specific primers. The restriction sites included in the primers were ECORV /NHEI, for VH and ECORV/SALI for VK. The primers sequences were
**For VH:**
Primer 1 (signal peptide):
   5'GGGGATATCCACCATGG(AG)ATG(CG)AGCTG(TG)GT(CA)AT(CG)CTCTT 3'
Primer 2 (CH1):
   5' GGGGCTAGCTGCAGAGACAGTGACCAGAGT 3'
**Para VK:**
Primer 1 (signal peptide):
   5' GGGGATATCCACCATGGAG(TA)CACA(GT)(TA)CTCAGGTCTTT(GA)T 3'
Primer 2 (Ck):
   5' AGCGTCGACTTACGTTT(TG)ATTTCCA(GA)CTT(GT)GTCCC 3'

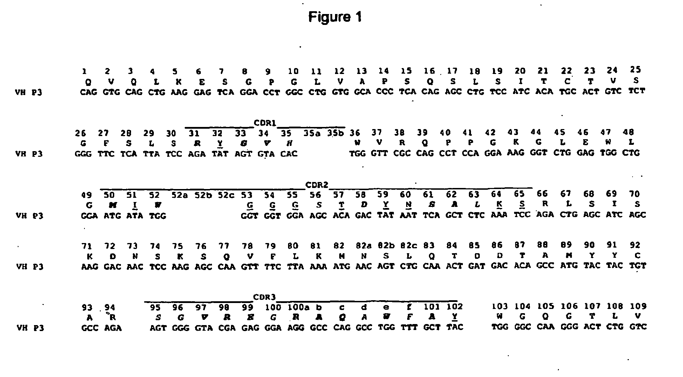

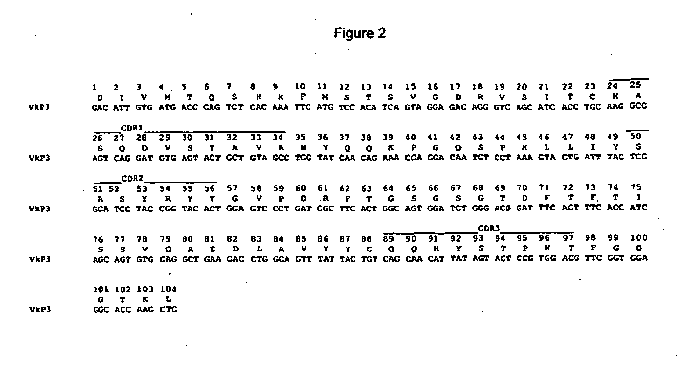

PCR products were cloned into TA vector (TA cloning kit, Invitrogen). Twelve independent clones were sequenced by the dideoxy method using T7 DNA Pol (Pharmacia). By homology search analysis it was determined the most homologous sequence group for VHP3 and VKP3. VHP3 and VKP3 sequences (Figures 1 and 2) have high homology with groups IB and V respectively according to Kabat's classification.

After digestion with the restriction enzymes ECORV and NHEI for VHP3 and with ECORV and SALI for VKP3, they were cloned in the expression vectors previously digested with the same enzymes, PAH4604 and PAG4622 for VH and VK respectively. These expression vectors were donated by Sherie Morrison (UCLA, California, USA), they are suitable for immunoglobulins expression in mammalian cells. The vector PAH 4604 have included the human constant region IgG1 and the PAG 4622 human (Coloma and collaborators (1992): Novel vectors for the expression of antibody molecules using variable regions generated by polymerase chain reaction, J. Immunol. Meth., 152: 89-104). The resultant constructs P3VH-PAH4604 and P3VK-PAG4622. NS-0 cells were transfected with 10 µg of P3VK-PAG4622, a clone expressing light chain was transfected with 10 ug P3VH-PAH4604, in both cases DNA is linearized with Pvul, ethanol precipitated and dissolved in 50 ul of PBS before transfection.

Approximately 10⁷ cells were harvested by centrifugation and resupended in 0.5 ml of PBS together with the digested DNA in an electroporation cuvette. After 10 minutes on ice, the cells were given a pulse of 200 volts and 960 uF and left in ice for a further 10 minutes. The cells were distributed into 96 wells plate with D'MEM F12 plus 10% fetal calf serum. Two or four days later, it is added selective medium (D'MEM F12 with mycophenolic acid 0,45 ug/mL or histidinol 10mM, respectively). Transfected clones were visible with the naked eyes 14 days later.

The presence of human antibody in the medium of wells containing transfected clones was measured by ELISA. Microtiter plate wells were coated with goat anti-human kappa light chain (for human kappa chain producing clones) or anti-human IgG (gamma chain specific) (for the complete antibody producing clones) antibodies. After washing with PBST (saline phosphate buffered solution containing 0.05% Tween 20), diluted culture medium of the wells containing transfectants was added to each Microtiter well for one hour at 37°C. The wells were washed with PBS-T and peroxidase of spicy radish-conjugated goat anti-human kappa light chain or alkaline fosfatase-conjugated goat anti-human IgG (gamma chain specific), were added and incubated at room temperature one hour. The wells were washed with PBS-T and substrate buffer containing *o*-phenylendiamine or *p*-nitrophenylphosphate, respectively, was added. After half hour absorbance at 492 or 405 nm respectively, was measured.

### Example 2. Obtaining different versions of the Humanized Antibody P3.

Murine VHP3 and VKP3 sequences (Figures 1 and 2) were compared with human sequences, Figures 3 and 4 shown the most homologous human sequences. Helical amphipatic regions or potential T cell epitopes were searched on murine P3 variable region sequences and according with the method a judiciously strategy for aminoacid replacements was established in order to break or humanize potential T cell epitopes into the murine sequences.

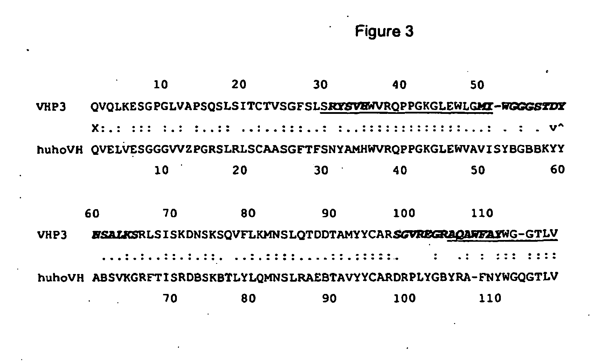

The analysis on VHP3 rendered (Figure 3) 2 amphipatic segments, the first one embraces CDR1, FR2 and some residues of the CDR2, the second one embraces the end of FR3 and CDR3. The main differences of murine sequence in comparison with the most homologous human sequence were founded in CDRs or residues involved with the three dimensional structure of the binding site. For that reason it was decided do not replace any aminoacid in murine VHP3.

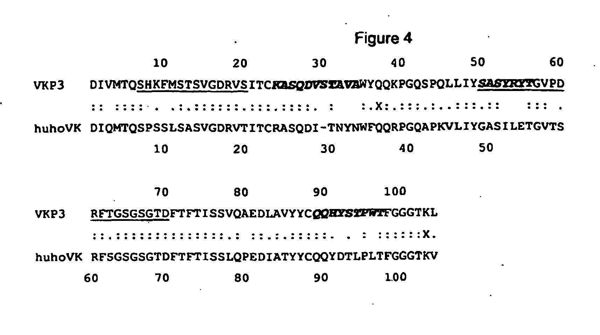

The analysis for VKP3 rendered also 2 amphipatic segments (Figure 4), the first segment embraces FR1, the second one embraces CDR2 and some residues of the FR3. It was decide to replace residues at positions 8,9,10,11 and 13 by residues at the same position in the most homologous human sequence. The amino acids His, Lys, Phe, Met and Thr were replaced by Pro, Ser, Ser, Leu, and Ala, respectively. The replacements were made by PCR overlapping (Kammann et collaborators (1989) Rapid insertional mutagénesis of DNA by polymerase chain reaction (PCR), Nucleic Acids Res., 17: 5404) using primers 1 and 2 and 3 and 4 whose sequences are the following:
Primer 1:
   5' ATGACCCAGTCTCCTTCTTCTCTTTCCGCGTCAGTAGGAGAC 3'
Primer 2:
   5' AGCGTCGACTTACGTTT(TG)ATTTCCA(GA)CTT(GT)GTCCC 3'
Primer 3:
   5' GTCTCCTACTGACGCGGAAAGAGAAGAAGGAGACTGGGTCAT 3'
Primer 4:
   5'GGGGATATCCACCATGGAG(TA)CACA(GT)(TA)CTCAGGTCTTT(GA)T 3'

The point mutations were verified by sequencing. The resultant construct was P3Vkhu and it was cloned in PAG 4622 expression vector. The resultant construct was P3VKhu-PAG4622.

To express the humanized antibody P3, NS-0 cells were transfected with P3VH-PAH4604 and P3VKhu-PAG4622

P3hu antibody was transfected following the same procedure described previously for the chimeric antibodies.

### Example 3: Biological activity of chimeric MAb P3

The specific binding to antigen measured by ELISA tested the biological activity of the Mab P3 chimeric.

For recombinant MAb P3, Microtiter plates were coated with GM3(NeuGc) ganglioside in methanol. After drying one hour at 37°C, unspecific binding was blockade with bovine sera albumin (BSA) 1% in Tris-HCl buffer, incubated for one hour at 37°C. The wells were washed with PBS and incubated for 1 hour at 37°C with purified recombinant Mab P3. The wells were washed with tris-HCl and a goat anti- human antibody conjugated with alkaline fosfatase was added and incubated at 37°C for one hour. Finally, the wells were washed with Tris-HCl and the substrate buffer containing *p*-nitrophenylphosphate was added. After half hour absorbance at 405 nm, was measured.

Mab T1 chimeric was used as negative control.

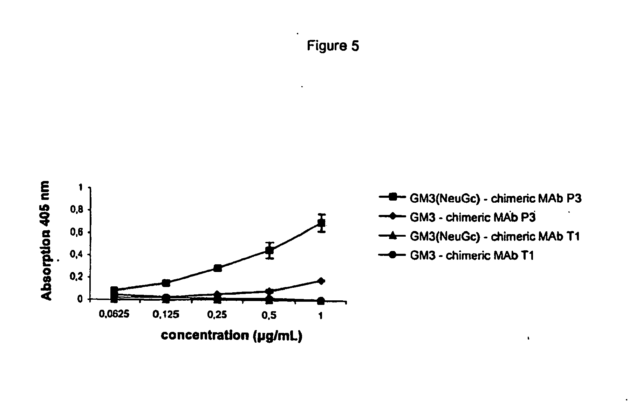

Figure 5 shows the specific binding of Mab P3 chimeric to the antigen.

### Example 4. Obtaining of chimeric MAb 1E10

The cDNA synthesis was obtained by a reaction with reverse transcriptase enzyme, starting with RNA from the hybridoma producing Mab 1E10, as described previously. The sequence of the specific primers used in this reaction is shown::
**ForVH:**
   5'GGGGCTAGCTGAGGAGACTGTGAGAGTGGT 3
**For VK:**
   5'GCGTCTAGAACTGGATGGTGGGAAGATGGA 3'
cDNA VH1E10 and cDNA VK1E10 were amplified by PCR using Taq Pol and specific primers.
**For VH:**
Primer 1 (signal peptide):
   5'GGGGATATCCACCATGG(AG)ATG(CG)AGCTG(TG)GT(CA)AT(CG)CTCTT 3'
Primer 2 (CH1):
   5'GGGGCTAGCTGAGGAGACTGTGAGAGTGGT 3'
**For VK:**
Primer 1 (signal peptide):
   5'GGGGTTAACCACCATGAGG(GT)CCCC(AT)GCTCAG(CT)T(CT)CT(TG)GG(GA)3'
Primer 2 (Ck):
   5'AGCGTCGACTTACGTTT(TG)ATTTCCA(GA)CTT(GT)GTCCC3'

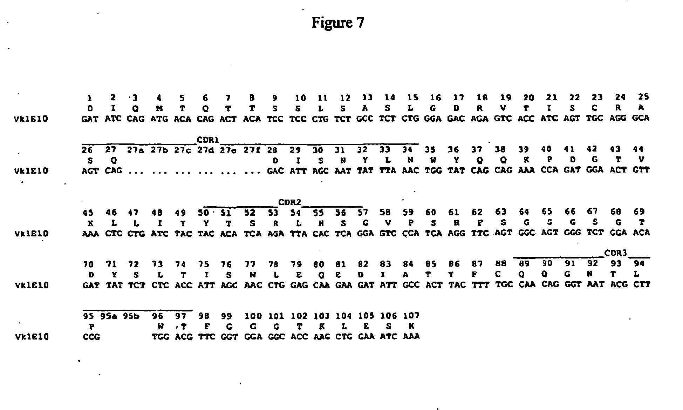

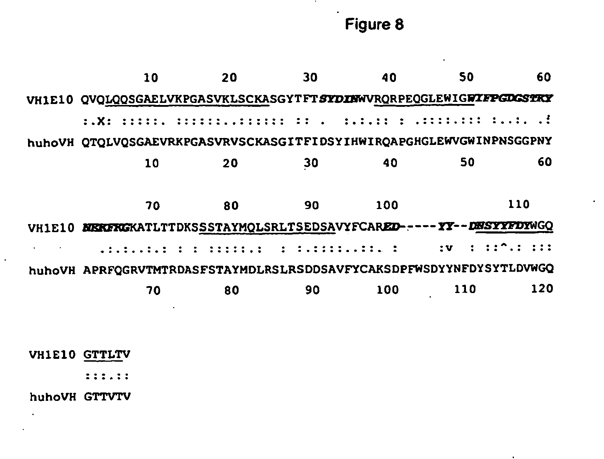

PCR products were cloned into TA vector (TA cloning kit, Invitrogen). Twelve independent clones were sequenced (Figures 7 and 8) by the dideoxy method using T7 DNA Pol (Pharmacia). By homology search analysis it was determined the most homologous sequence group for VH1E10 and VK1E10. VH1E10 and VK1E10 sequences have high homology with groups miscellaneous and V respectively according to Kabat's classification.

After digestion with the restriction enzymes ECORV and NHEI for VH1E10 and with HincII and SALI for VK1E10, they were cloned in the expression vectors previously digested with appropriated enzymes, PAH4604 and PAG4622 for VH and VK respectively. These expression vectors were donated by Sherie Morrison (UCLA, California, USA), they are suitable for immunoglobulins expression in mammalian cells. The vector PAH 4604 have included the human constant region IgG1 and the PAG 4622 human (Coloma et collaborators (1992): Novel vectors for the expression of antibody molecules using variable regions generated by polymerase chain reaction, J. Immunol. Meth., 152: 89-104). The resultant constructs 1E10VH-PAH4604 and 1E10VK-PAG4622.

NS-0 cells were transfected with 10 µg of 1E10VK-PAG4622, a clone expressing light chain was transfected with 10 ug 1E10VH-PAH4604, in both cases DNA is linearized with Pvul, ethanol precipitated and dissolved in 50 ul of PBS before transfection.

Approximately 10⁷ cells were harvested by centrifugation and resupended in 0.5 ml of PBS together with the digested DNA in an electroporation cuvette. After 10 minutes on ice, the cells were given a pulse of 200 volts and 960 uF and left in ice for a further 10 minutes. The cells were distributed into 96 wells plate with D'MEM F12 plus 10% fetal calf serum. Two or four days later, it is added selective medium (D'MEM F12 with mycophenolic acid 0,45 ug/mL or histidinol 10mM, respectively). Transfected clones were visible with the naked eyes 14 days later.

The presence of human antibody in the medium of wells containing transfected clones was measured by ELISA. Microtiter plate wells were coated with goat anti-human kappa light chain (for human kappa chain producing clones) or anti-human IgG (gamma chain specific) (for the complete antibody producing clones) antibodies. After washing with PBST (phosphate buffered saline containing 0.05% Tween 20), diluted culture medium of the wells containing transfectants was added to each Microtiter well for one hour at 37°C. The wells were washed with PBS-T and peroxidase of spicy radish-conjugated goat anti-human kappa light chain or alkaline fosfatase-conjugated goat anti-human IgG (gamma chain specific), were added and incubated at room temperature one hour. The wells were washed with PBS-T and substrate buffer containing *o-*phenylendiamine or *p*-nitrophenylphosphate, respectively, was added. After half hour absorbance at 492 or 405 nm respectively, was measured.

### Example 5. Obtaining different versions of the Humanized Antibody 1E10

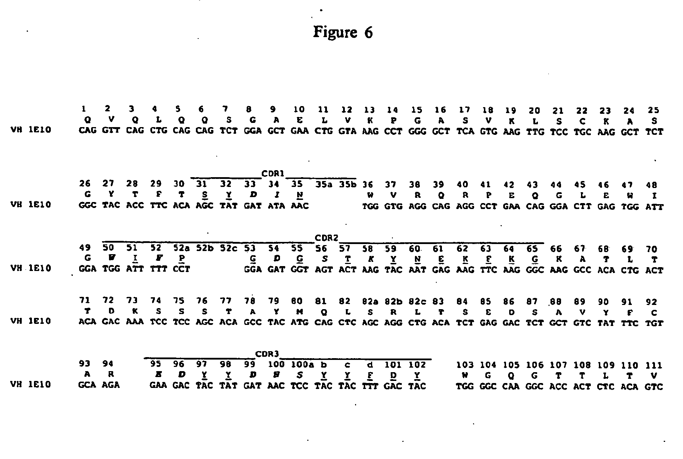

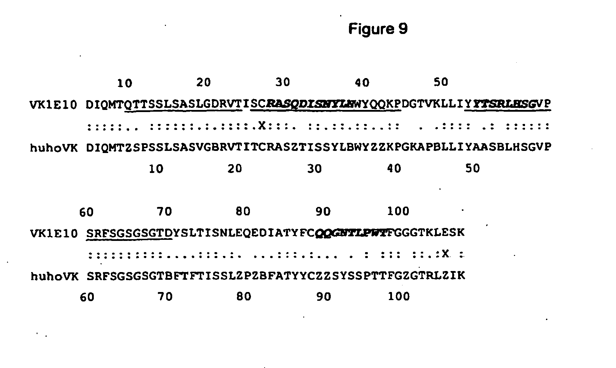

Murine VH1E10 VK1E10 sequences (Figures 6 and 7) were compared with human sequences, Figures 8 and 9 shown the most homologous human sequences. Helical amphipatic regions or potential T cell epitopes were searched on murine 1E10 variable region sequences and according with the method a judiciously strategy for aminoacid replacements was established in order to break or humanize potential T cell epitopes into the murine sequences

The analysis on VH1E10 rendered (Figure 8) 3 amphipatic segments, the first one embraces FR1, the second one embraces FR2, the third one embraces FR3. It was decide to replace residues at positions 5, 40, 42 and 87 87 (83 according to Kabat's numbering) by residues at the same position in the most homologous human sequence. The amino acids GIn, Arg, Glu and Thr were replaced by Val, Ala, Gly and Arg, respectively.

The replacements were made by PCR overlapping (Kammann et collaborators (1989) Rapid insertional mutagenesis of DNA by polymerase chain reaction (PCR), Nucleic Acids Res., 17: 5404) using different set of primers.

Primers for mutation at position 5 of the heavy chain were 1 and 2 and 3 and 4 whose sequences are the following:
Primer 1:
   5' CAGGTTCAGCTGGTGCAGTCTGGAGCT 3'
Primer 2:
   5'GGGGCTAGCTGAGGAGACTGTGAGAGTGGT3'
Primer 3:
   5' AGCTCCAGACTGCACCAGCTGAACCTG 3'
Primer 4:
   5'GGGGATATCCACCATGG(AG)ATG(CG)AGCTG(TG)GT(CA)AT(CG)CTCTT 3'

After checking by sequence the point mutation at position 5, mutations at positions 40 and 42 were introduced.

Primer for mutations at positions 40 and 42 of the heavy chain:
Primer 1:
   5' TGGGTGAGGCAGGCGCCTGGGCAGGGACTTGAG 3'
Primer 2:
   5'GGGGCTAGCTGAGGAGACTGTGAGAGTGGT3'
Primer 3:
   5' CTCAAGTCCCTGCCCAGGCGCCTGCCTCACCCA 3'
Primer 4:
   5'GGGGATATCCACCATGG(AG)ATG(CG)AGCTG(TG)GT(CA)AT(CG)CTCTT 3'

After checking by sequence the point mutation at positions 40 and 42, mutation at positions 87 (83 according to Kabat's numbering) was introduced.

Primer for mutations at position 87 (83 according to Kabat's numbering) of the heavy chain:
Primer 1:
   5' CTCAGCAGGCTGCGGTCTGAGGACTCT 3'
Primer 2:
   5'GGGGCTAGCTGAGGAGACTGTGAGAGTGGT3'
Primer 3:
   5' AGAGTCCTCAGACCGCAGCCTGCTGAG 3'
Primer 4:
   5'GGGGATATCCACCATGG(AG)ATG(CG)AGCTG(TG)GT(CA)AT(CG)CTCTT 3'

Other replacements were not made because residues were involved in the three dimensional structure of the binding site.

The point mutations were verified by sequencing. The resultant construct was 1E10VHhu and it was cloned in PAH4604 expression vector. The resultant construct was 1E10VH-PAH4604.

The analysis for VK1E10 rendered also 3 amphipatic segments (Figure 9), the first segment embraces FR1, the second one embraces CDR1 and the thirst one embraces FR3. It was decide to replace residues at positions 7,8 and 15 by residues at the same position in the most homologous human sequence. The amino acids Thr, Thr and Leu were replaced by Ser, Pro and Val, respectively. The replacements were made by PCR overlapping (Kammann et collaborators (1989) Rapid insertional mutagenesis of DNA by polymerase chain reaction (PCR), Nucleic Acids Res., 17: 5404) using primers 1 and 2 and 3 and 4 whose sequences are the following:
Primers for mutation at positions 7, 8 and 15 of the light chain:
   Primer 1:
      5'CAGATGACACAGTCTCCTTCCTCCCTGTCTGCCTCTGTGGGAGACAGAGTC 3'
   Primer 2:
      5'AGCGTCGACTTACGTTT(TG)ATTTCCA(GA)CTT(GT)GTCCC 3'
   Primer 3:
      5'GACTCTGTCTCCCACAGAGGCAGACAGGGAGGAAGGAGACTGTGTCATCTG 3'
   Primer 4:
      5'GGGGTTAACCACCATGAGG(GT)CCCC(AT)GCTCAG(CT)T(CT)CT(TG)GG(GA) 3'

The point mutations were verified by sequencing. The resultant construct was 1E10Vkhu and it was cloned in PAG 4622 expression vector. The resultant construct was 1E10VKhu-PAG4622. To express the humanized antibody 1 E10, NS-0 cells were transfected with 1 E10VHhu-PAH4604 and 1E10VKhu-PAG4622.

1E10hu antibody was transfected following the same procedure described previously for the chimeric antibodies.

### Example 6: Biological activity of chimeric MAb1E10.

The specific binding to antigen measured by ELISA tested the biological activity of the Mab 1E10 chimeric.

For recombinant MAb 1E10, Microtiter plates were coated with Mab P3. After washing with PBST (saline phosphate buffered solution containing 0.05% Tween 20), unspecific binding was blockade with bovine sera albumin (BSA) 1% in PBST, incubated for one hour at 37°C. The wells were washed and incubated for 1 hour at 37°C with purified recombinant Mab 1E10. The wells were washed with PBST and a goat anti- human antibody conjugated with alkaline fosfatase was added and incubated at 37°C for one hour. Finally, the wells were washed with PBST and the substrate buffer containing *p*-nitrophenylphosphate was added. After half hour absorbance at 405 nm respectively, was measured.

Mab C5 chimeric was used as negative control.

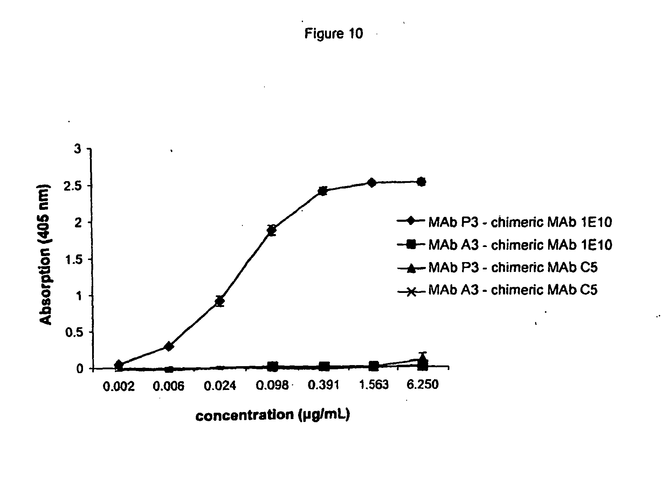

Figure 10 shows the specific binding of Mab 1E10 chimeric to Mab P3.

### Brief description of the figures:

**Figure 1****:** VHP3 DNA and deduced amino acid sequences. Sequences are aligned according Kabat's numbering (Kabat and collaborators (1991), Sequences of proteins of immunological interest, Fifth Edition, National Institute of Health), CDRs appeared marked with dotted lines.
**Figure 2****:** VKP3 DNA and deduced amino acid sequences. Sequences are aligned according Kabat's numbering (Kabat and collaborators (1991), Sequences of proteins of immunological interest, Fifth Edition, National Institute of Health), CDRs appeared marked with dotted lines.
**Figure 3****:** VHP3 was aligned with the most homologous human sequence. Amphipatic segments are underlined and CDRs in bold.
**Figure 4****:** VKP3 was aligned with the most homologous human sequence. Amphipatic segments are underlined and CDRs in bold.
**Figure 5****:** Specific binding to GM3(NeuGc) by chimeric Mab P3. Different concentrations of Mab P3 an MAb T1 (negative control) were tested by ELISA. Microtiter plates were coated with GM3(NeuGc) and GM3(NeuAc) (negative control) ganglioside in methanol and specific binding was measured.
**Figure 6****:** VH1E10 DNA and deduced amino acid sequences. Sequences are aligned according Kabat's numbering (Kabat and collaborators (1991), Sequences of proteins of immunological interest, Fifth Edition, National Institute of Health), CDRs appeared marked with dotted lines.
**Figure 7****:** VK1E10 DNA and deduced amino acid sequences. Sequences are aligned according Kabat's numbering (Kabat and collaborators (1991), Sequences of proteins of immunological interest, Fifth Edition, National Institute of Health), CDRs appeared marked with dotted lines.
**Figure 8****:** VH1E10 was aligned with the most homologous human sequence. Amphipatic segments are underlined and CDRs in bold.
**Figure 9****:** VK1E10 was aligned with the most homologous human sequence. Amphipatic segments are underlined and CDRs in bold.
**Figure 10****:** Specific binding murine Mab P3 by chimeric Mab 1E10. Different concentrations of Mab 1E10 an MAb C5 (negative control) were tested by ELISA. Microtiter plates were coated with Mab P3 and Mab A3 (negative control) and specific binding was measured.

## Claims

1. A chimeric monoclonal antibody derived from the murine anti-idiotypic monoclonal antibody 1E10 that recognizes murine MAb P3 and produced by the hybridoma cell line with deposit number ECACC 97112901, wherein the constant region of the heavy chain comprises the amino acid sequence of gamma-1 chain and the constant region of the light chain comprises the amino acid sequence of a kappa chain, both derived from human immunoglobulins and wherein the hypervariable domains of its heavy and light chains comprise the following sequences:
**HEAVY CHAIN**
CDR1: SYDIN
CDR2: WIFPGDGSTKYNEKFKG
CDR3: EDYYDNSYYFDY
**LIGHT CHAIN**
CDR1:RASQDISNYLN
CDR2: YTSRLHSG
CDR3: QQGNTLPWT
and wherein the framework regions (FRs) of its heavy and light chains comprise the following sequences:
**HEAVY CHAIN**
FR1: QVQLQQSGAELVKPGASVKLSCKASGYTFT
FR2: WVRQRPEQGLEWIG
FR3: KATLTTDKSSSTAYMQLSRLTSEDSAVYFCAR
FR4: WGQGTTLTV
**LIGHT CHAIN**
FR1: DIQMTQTTSSLSASLGDRVTISC
FR2: WYQQKPDGTVKLLIY
FR3: VPSRFSGSGSGTDYSLTISNLEQEDIATYFC
FR4: FGGGTKLESK

2. A monoclonal antibody according to claim 1, wherein for its humanization and preserving the binding properties of the antigen, the variable region of the antibody includes the following substitutions:
**LIGHT CHAIN:**
Position 7: Thr by Ser
Position 8: Thr by Pro
Position 15: Leu by Val
**HEAVY CHAIN**
Position 5: GIn by Val
Position 40: Arg by Ala
Position 42: Glu by Gly
Position 87 (83 according to Kabat's numbering): Thr by Arg

3. A cell line that produces any of the monoclonal antibodies of claims 1 or 2.

4. A pharmaceutical composition for use in the treatment of malignant tumors of breast and melanomas, their metastases and relapses which comprises any of the monoclonal antibodies of claims 1 or 2.

5. A pharmaceutical composition for use in "*in vivo"* localization and identification of malignant tumors of breast and melanomas, their metastases and relapses, comprising any of the monoclonal antibodies of claims 1 or 2.

6. Use of the monoclonal antibody of any one of the claims 1 or 2 for the manufacture of a medicament for the treatment of malignant tumors of breast and melanomas, their metastases and relapses.

7. A monoclonal antibody of any one of the claims 1 or 2, for use in the treatment of malignant tumors of breast and melanomas, their metastases and relapses.

## Patentansprüche

1. Chimärer monoklonaler Antikörper, der von einem murinen anti-ideotypischen monoklonalen Antikörper 1E10 abgeleitet ist, welcher den murinen MAb P3 erkennt und von der Hybridom-Zelllinie mit der Hinterlegungsnummer ECACC 97112901 produziert wird, wobei die konstante Region der schweren Kette die Aminosäuresequenz der Gamma-1-Kette umfasst und die konstante Region der leichten Kette die Aminosäuresequenz der Kappa-Kette umfasst, wobei beide von humanen Immunglobulinen abgeleitet wurden, und wobei die hypervariablen Regionen seiner schweren und leichten Ketten die folgenden Sequenzen umfassen:
**SCHWERE KETTE:**
CDR1: SYDIN
CDR2: WIFPGDGSTKYNEKFKG
CDR3: EDYYDNSYYFDY
**LEICHTE KETTE:**
CDR1: RASQDISNYLN
CDR2: YTSRLHSG
CDR3: QQGNTLPWT
und wobei die Gerüstregionen (framework regions, FRs) seiner schweren und leichten Kette die folgenden Sequenzen umfassen:
**SCHWERE KETTE:**
FR1: QVQLQQSGAELVKPGASVKLSCKASGYTFT
FR2: WVRQRPEQGLEWIG
FR3: KATLTTDKSSSTAYMQLSRLTSEDSAVYFCAR
FR4: WGQGTTLTV
**LEICHTE KETTE:**
FR1: DIQMTQTTSSLSASLGDRVTISC
FR2: WYQQKPDGTVKLLIY
FR3: VPSRFSGSGSGTDYSLTISNLEQEDIATYFC
FR4: FGGGTKLESK

2. Monoklonaler Antikörper nach Anspruch 1, wobei für seine Humanisierung und für die Erhaltung der Bindungseigenschaften des Antigens die variable Region des Antikörpers die folgenden Substitutionen umfasst:
**LEICHTE KETTE:**
Position 7: Thr durch Ser
Position 8: Thr durch Pro
Position 15: Leu durch Val
**SCHWERE KETTE:**
Position 5: Gln durch Val
Position 40: Arg durch Ala
Position 42: Glu durch Gly
Position 87 (83 nach Kabat-Nummerierung): Thr durch Arg.

3. Zelllinie, die einen der monoklonalen Antikörper nach Anspruch 1 oder 2 erzeugt.

4. Pharmazeutische Zusammensetzung zur Verwendung bei der Behandlung von malignen Brust-Tumoren und Melanomen, deren Metastasen und Rezidiven, die einen der monoklonalen Antikörper nach Anspruch 1 oder 2 umfasst.

5. Pharmazeutische Zusammensetzung zur Verwendung bei der "in vivo"-Lokalisierung und Identifizierung von malignen Brust-Tumoren und Melanomen, deren Metastasen und Rezidiven, die einen der monoklonalen Antikörper nach Anspruch 1 oder 2 umfasst.

6. Verwendung des monoklonalen Antikörpers nach einem der Ansprüche 1 oder 2 zur Herstellung eines Medikamentes zur Behandlung von malignen Brust-Tumoren und Melanomen, deren Metastasen und Rezidiven.

7. Monoklonaler Antikörper nach einem der Ansprüche 1 oder 2 zur Verwendung bei der Behandlung von malignen Brust-Tumoren und Melanomen, deren Metastasen und Rezidiven.

## Revendications

1. Anticorps monoclonal chimérique dérivé de l'anticorps monoclonal anti-idiotypique murin 1E10 qui reconnaît l'Acm P3 murin et produit par la lignée cellulaire d'hybridome portant le numéro de dépôt ECACC 97112901, dans lequel la région constante de la chaîne lourde comprend la séquence d'acides aminés de la chaîne gamma 1 et la région constante de la chaîne légère comprend la séquence d'acides aminés d'une chaîne kappa, dérivées l'une et l'autre d'immunoglobulines humaines, et dans lequel les domaines hypervariables de ses chaînes lourde et légère comprennent les séquences suivantes :
**CHAÎNE LOURDE**
CDR1 : SYDIN
CDR2 : WIFPGDGSTKYNEKFKG
CDR3 : EDYYDNSYYFDY
**CHAÎNE LÉGÈRE**
CDR1 : RASQDISNYLN
CDR2 : YTSRLHSG
CDR3 : QQGNTLPWT
et dans lequel les régions charpentes (FR) de ses chaînes lourde et légère comprennent les séquences suivantes :
**CHAÎNE LOURDE**
FR1 : QVQLQQSGAELVKPGASVKLSCKASGYTFT
FR2 : WVRQRPEQGLEWIG
FR3 : KATLTTDKSSSTAYMQLSRLTSEDSAVYFCAR
FR3 : WGQGTTLTV
**CHAÎNE LÉGÈRE**
FR1 : DIQMTQTTSSLSASLGDRVTISC
FR2 : WYQQKPDGTVKLLIY
FR3 : VPSRFSGSGSGTDYSLTISNLEQEDIATYFC
FR4 : FGGGTKLESK

2. Anticorps monoclonal selon la revendication 1, dans lequel, pour son humanisation et pour préserver 1 es propriétés de liaison de l'antigène, la région variable de l'anticorps comprend les substitutions suivantes :
**CHAÎNE LÉGÈRE :**
Position 7 : Thr par Ser
Position 8 : Thr par Pro
Position 15 : Leu par Val
**CHAÎNE LOURDE**
Position 5 : Gln par Val
Position 40 : Arg par Ala
Position 42 : Glu par Gly
Position 87 (83 suivant la numérotation de Kabat) : Thr par Arg

3. Lignée cellulaire produisant l'un quelconque des anticorps monoclonaux selon la revendication 1 ou la revendication 2.

4. Composition pharmaceutique destinée à être utilisée dans le traitement de tumeurs mammaires malignes et de mélanomes, de leurs métastases et de leurs récidives, comprenant l'un quelconque des anticorps monoclonaux selon la revendication 1 ou la revendication 2.

5. Composition pharmaceutique destinée à être utilisée dans la localisation et l'identification *in vivo* de tumeurs mammaires malignes et de mélanomes, de leurs métastases et de leurs récidives, comprenant l'un quelconque des anticorps monoclonaux selon la revendication 1 ou la revendication 2

6. Utilisation de l'anticorps monoclonal selon l'une quelconque des revendications 1 ou 2 pour fabriquer un médicament destiné au traitement de tumeurs mammaires malignes et de mélanomes, de leurs métastases et de leurs récidives.

7. Anticorps monoclonal selon l'une quelconque des revendications 1 ou 2, destiné à être utilisé dans le traitement de tumeurs mammaires malignes et de mélanomes, de leurs métastases et de leurs récidives.
